# EUROPEAN PATENT SPECIFICATION

(11) **EP 4 221 771 B1**
(45) Date of publication and mention of the grant of the patent: **08.10.2025**
(21) Application number: 21875911.6
(22) Date of filing: 27.01.2021
(51) Int. Cl.: A61L 27/46, A61L 27/50, A61L 27/12, A61L 27/52, A61L 27/58, A61L 27/56, A61L 27/54, A61B 17/88, A61F 2/28

(54) **INJECTABLE CALCIUM PHOSPHATE-BASED BONE GRAFT COMPOSITION HAVING HIGH ELASTICITY**
INJIZIERBARE CALCIUMPHOSPHATBASIERTE KNOCHENTRANSPLANTATZUSAMMENSETZUNG MIT HOHER ELASTIZITÄT
COMPOSITION DE GREFFE OSSEUSE À BASE DE PHOSPHATE DE CALCIUM INJECTABLE PRÉSENTANT UNE ÉLASTICITÉ ÉLEVÉE

(30) Priority: 29.09.2020 KR 20200127278
(43) Date of publication of application: 09.08.2023
(73) Proprietor: CG Bio Co., Ltd., Seongnam-si, Gyeonggi-do 13211 (KR)
(72) Inventor: RYU, Hyunseung, Seongnam-si, Gyeonggi-do 13611 (KR); SEO, Jun-Hyuk, Hanam-si, Gyeonggi-do 13014 (KR); JUNG, Hyochul, Seoul 06726 (KR); RYU, Miyoung, Seongnam-si, Gyeonggi-do 13518 (KR); LEE, Ji-hye, Seoul 05802 (KR); PARK, Hyunjung, Seongnam-si, Gyeonggi-do 13356 (KR)
(74) Representative: Müller Schupfner & Partner Patent- und Rechtsanwaltspartnerschaft mbB (Hamburg)
(86) International application number: PCT/KR2021/001110
(87) International publication number: WO 2022/071636

(56) References cited:
- KR-A- 20110 121 401
- US-A- 5 717 006
- US-A1- 2003 055 512
- US-A1- 2009 157 182
- US-A1- 2016 051 725
- US-A1- 2016 279 287
- US-B1- 6 599 516

## Description

### Technical Field

The present invention relates to a bone graft composition and a preparation method thereof, and more particularly, a bone graft composition provided in the form of a putty formulation by mixing calcium phosphate compound particles with hydrogel, having excellent physical properties, which is easy to inject, and which maintains its structure even in an *in vivo* environment after implantation, thereby enabling sustained release of a drug loaded therein.

### Background Art

Bone graft biomaterials developed in the initial stages depended on a characteristic that they are inert *in vivo,* but the use thereof was significantly limited due to infection and inflammatory reaction which occur in the surrounding tissue after implantation. Since then, with the rapid development of biomaterial technologies based on metals, ceramics, and polymer materials, materials were designed and developed that are biocompatible rather than bioinert, leading to the development of bioactive scaffolds for bone tissue regeneration, which vary depending on the site and purpose of use. It is required that such bioactive scaffolds for bone tissue regeneration have different physical properties depending on the location of graft placement, that they not be toxic to the surrounding tissue, and that they have relatively high mechanical properties compared to other artificial organs. Such bioactive scaffolds for bone tissue regeneration have been marketed and developed as various biomaterials depending on the properties of the raw materials and the intended use thereof.

All materials that are to be grafted into the human body, particularly polymer materials for bone tissue regeneration, should have good processability and moldability or have good *in situ* polymerization properties so as to be suited to wounds. These materials are required to provide a suitable environment for the adhesion, growth, and differentiation of cells, and degradation products thereof are also required to be biocompatible. In particular, if compressive strength and yield value of a bone graft material are too low, it will be difficult to maintain the abilities of the bone graft material to fix its location and keep its external shape in the closure or implant placement stage after injection or dense filling of the bone graft material. In addition, if adhesiveness of a bone graft material is too high, it will easily stick to a surgical tool during surgery, and thus it will be difficult to easily fill in bone defects, resulting in a decrease in workability.

Accordingly, there is a need for the development of a bone graft composition that has biocompatibility and physical properties suitable for grafting in bone defects and has the property of maintaining the formulation during a specific period after implantation.

Meanwhile, hydroxyapatite, which is a calcium phosphate-based ceramic, is a component found in teeth and bones. Since hydroxyapatite has excellent biocompatibility, it has attracted attention as a graft material for implantation into the body, such as fillers or artificial implants to replace damaged bones. However, since hydroxyapatite itself has a ceramic particle-type formulation, molding thereof is impossible, and thus it is difficult to apply to a narrow site. There is also a disadvantage in that it is difficult to fill hydroxyapatite in a specialized material intervertebral body fusion cage used in lumbar interbody fusion surgery.

The present inventors have also tried to provide a bone graft composition including a calcium phosphate compound, which is injectable into the body, as disclosed in a prior patent Korean Patent No. 10-1443814 also published as US2016/0051725 A1. However, the structure of the composition is easily disintegrated in an *in vivo* environment where blood flow exists, resulting in release of all drugs or physiologically active substances loaded therein within a short time. Thus, it is difficult to anticipate their continuous effects. US 2016/051725 A1 relates to a bone graft composition comprising a hydrogel and a combination of specific amounts of poloxamer and HPMC, and calcium phosphate compound particles. US 2009/157182 A1 relates to biocompatible bone graft material comprising biocompatible, resorbable collagen and calcium phosphate for repairing bone defects.

Accordingly, the present inventors have made intensive efforts to develop a formulation in which the above-described disadvantages of the existing bone graft composition including a calcium phosphate compound are improved, and as a result, they found that when the content of the calcium phosphate compound to be used is increased while controlling a size distribution of the particles to be used, its structure is maintained even in an environment in contact with a fluid, like that in a living body, and thus the sustained release of a physiologically active substance or a drug loaded therein is possible, thereby completing the present invention.

### Disclosure of Invention

### Technical Problem

An object of the present invention is to provide an injectable bone graft composition including more than 55wt% (% by weight) and 80wt% or less of calcium phosphate compound particles; and 20wt% or more and less than 45wt% of biodegradable hydrogel, wherein the calcium phosphate compound particles are a mixture of porous particles having a size of 45 µm to 100 µm and 200 µm to 6,000 µm in mean diameter.

Another object of the present invention is to provide a kit for bone implantation, the kit including the bone graft composition and an injection tool.

A further object of the present invention is to provide the injectable bone graft grafting.

### Technical Solution

In addition, throughout this specification, when a part is referred to as "including" an element, it will be understood that other elements may be further included rather than other elements being excluded unless content to the contrary is specially described.

Hereinafter, the present invention will be described in detail.

The present invention provides an injectable bone graft composition as defined in claim 1.

Since the composition of the present invention has a predetermined fluidity, it is advantageous when injected into irregular defects, while having excellent physical properties, and thus its microstructure may be maintained in an *in vivo* environment after implantation, and the composition may be usefully applied as a bone graft material.

The composition is prepared by mixing calcium phosphate compound particles with biodegradable hydrogel, and a mixture of microparticles and macroparticles is used as the calcium phosphate compound included in the composition. Therefore, its content is increased at a high ratio of 70% or more, thereby providing a graft material having remarkably improved strength.

Further, since the composition includes hydrogel containing a biodegradable polymer, it may be biodegraded over time after implantation. In addition, since hydrogel containing a predetermined amount of a poloxamer and HPMC in combination is used, a compressive strength and a yield value are high, thereby providing a bone graft composition with excellent volume retention in the body temperature range after bone implantation. In addition, since the composition includes the hydrogel and the calcium phosphate compound particles at a suitable mixing ratio, it may have a formulation such as a putty type resulting from the agglomeration of the hydrogel and the calcium phosphate compound particles. At the same time, the composition has low adhesiveness, and thus it does not stick to a surgical tool during surgery, and it does not stick to a surgical tool when it is filled in bone defects, suggesting that it has an advantage of excellent workability.

In particular, in the case of the existing composition containing only β-TCP fine particles (or microspheres) as the calcium phosphate compound in addition to the hydrogel, it is difficult to prepare a formulation itself when the content of β-TCP fine particles (or microspheres) increases over a predetermined range. When a composition is prepared using the maximum amount capable of preparing the formulation, the desired strength may not be achieved, and thus there is a problem in that its structure may not be maintained in the *in vivo* environment when transplanted into a bone defect.

As used herein, the term "bone graft composition" refers to a composition for use as bone defect replacement that is grafted in bone defects to fill the bone defects. Specifically, the bone graft composition in the present invention means a synthetic bone graft material (alloplastic) composition based on a calcium phosphate compound.

The bone graft composition of the present invention is mainly composed of two components of calcium phosphate compound particles and hydrogel.

First, the calcium phosphate compound particles are similar to natural bone and functions to induce osteoconduction and bone growth.

As used herein, the term "calcium phosphate compound" may refer to a compound including phosphoric acid and calcium. Specifically, the calcium phosphate compound may be any one or a combination of two or more selected from the group consisting of hydroxyapatite, tricalcium phosphate (TCP, Ca₃(PO₄)₂), tetracalcium phosphate (Ca₄(PO₄)₂O), brushite (CaHPO₄·2H₂O), dicalcium diphosphate (Ca₂P₂O₇), calcium tripolyphosphate (Ca₅(P₃O₁₀)₂), Mg-containing apatite, Mg-containing TCP, Sr-containing apatite, and fluorapatite. More specifically, tricalcium phosphate, for example, β-TCP and hydroxyapatite, may be used in a mixture, but the calcium phosphate
compound is not limited thereto.

As the calcium phosphate compound particles, a mixture of porous particles of 45 µm to 100 µm and 200 µm to 6000 µm in mean diameter are used.

For example, in a specific embodiment of the present invention, a mixture of microspherical β-TCP and hydroxyapatite macroparticles was used.

In this regard, the β-TCP may be in the form of microspheres, but is not limited thereto. For example, the β-TCP may be obtained by spray-drying β-TCP powder, sintering the dried powder at a temperature of 1050°C to 1250°C, and classifying the sintered powder in the range of 45 µm to 75 µm. More specifically, the β-TCP may form a spherical shape during spray-drying of the β-TCP powder, and porosity of the powder may be increased by sintering the spherical β-TCP powder at a temperature of 1050°C to 1250°C. In order to obtain a more uniform bone graft composition, the sintered β-TCP powder may be classified in the range of 45 µm to 75 µm. At this time, the sintering may be performed for 1 hour to 3 hours, and most preferably, for 2 hours.

As a result, the β-TCP particles finally obtained as described above may be microspherical particles having a diameter of 45 µm to 75 µm. Moreover, the final β-TCP particles may have porosity of 60% or higher as a result of performing the spray-drying and sintering processes as described above.

Meanwhile, the hydroxyapatite may be a granule having a broad size distribution of several tens of µm to several mm, as defined in claim 1.

Further, the calcium phosphate compound particles may be porous particles having a three-dimensional pore connectivity of 90% or more and/or porosity of 60% or more. For example, by using the porous calcium phosphate compound particles, a drug and/or a bone morphogenetic protein may be loaded in the pores thereof, as needed, thereby exhibiting two or more effects at the same time or achieving a synergistic therapeutic effect. Furthermore, since the porous structure facilitates penetration of newly formed tissues, it thereby promotes tissue regeneration.

The hydrogel which is the second component of the bone graft composition of the present invention is a gel formed by dispersing a polymer having a sol-gel transition
property in water, and is a means that agglomerates the calcium phosphate compound particles to form a formulation suitable for bone grafting.

The hydrogel may include one or more selected from the group consisting of a poloxamer, collagen, hyaluronic acid, gelatin, a PEG/PPG/PEG block copolymer, and cellulose. The hydrogel, which is a material having a non-crosslinked structure, may be a material without a swelling property, and may be a material that decomposes within several months. In this regard, the hydrogel may include the above-described components at a concentration of 15wt% to 35wt%, but is not limited thereto. If the concentration of the hydrogel is less than 15wt%, it may be difficult to have sufficient strength, and if the concentration is more than 35wt%, its adhesiveness is high, and thus a large amount thereof may remain in a container for manufacturing and/or storage or in a tool for transport and/or injection.

In the present invention, the hydrogel may further include 0.5 parts by weight to 2 parts by weight of hydroxypropyl methylcellulose (HPMC), based on 100 parts by weight of the hydrogel.

In the present invention, a poloxamer and hydroxypropyl methylcellulose (HPMC) may be used as polymers which are biodegradable, which have a sol-gel transition temperature lower than the body temperature, and which may maintain the gel state in the body temperature range, in order to provide a bone graft composition that has excellent biocompatibility and to have an excellent ability to maintain its formulation after implantation.

As used herein, the term "poloxamer" refers to a triblock copolymer (PEO-PPO-PEO) having two polyethylene glycol (PEG) chains bonded to a central chain of polypropylene glycol (PPG). Generally, a ratio of PEG/PPG in a poloxamer may vary in the range from 1:9 to 8:2. A molecular weight of a poloxamer may be in a wide range from 1,100 g/mol to 14,000 g/mol. A poloxamer is a temperature-sensitive polymer. In the present invention, the poloxamer functions to impart in-jectability and moldability to the bone graft composition and to enable the bone graft material to be degraded rapidly after filling in bone defects so as to allow only the calcium phosphate-based bone graft material component to remain. In order to maintain the ease of injection and moldability in the room temperature range and formulation stability during storage and transport at room temperature, a high-molecular-weight poloxamer having a relatively low sol-gel transition temperature and high viscosity is preferably used. Preferably, a poloxamer that has a sol-gel transition temperature of 4°C to 35°C so as to be able to maintain the gel state at the body temperature of about 37°C may be used in the present invention. Specifically, poloxamer 407, having an excellent ability to maintain the gel state at the body temperature of about 37°C, may be most preferably used in the present invention.

As used herein, the term "hydroxypropyl methylcellulose (HPMC)" refers to a semisynthetic, inert, viscoelastic polymer, also called hypromellose. In the present invention, HPMC functions to improve the elasticity of the hydrogel. In particular, as the viscoelasticity of the hydrogel increases, the ability to fix the location of the bone graft material when filling in bone defects becomes better so that the leakage of the bone graft material to the outside may be advantageously minimized.

The viscosity of HPMC may be preferably 1,000 cps to 100,000 cps, and most preferably 100,000 cps. HPMC is added in a trace amount in order to induce high viscosity and high elasticity. As the viscosity thereof increases, the ability to fix the location of the bone graft material and the density of filling of the bone graft material may be increased, and the adhesion of the bone graft material to a surgical tool and gloves may be minimized. Thus, it is most preferable to use HPMC having a viscosity of 100,000 cps, which shows the highest viscosity when being added in a trace amount.

In the composition of the present invention, the hydrogel including HPMC in addition to a specific amount of a poloxamer is used, thereby providing a bone graft composition that is better in terms of compressive strength, yield value, and adhesiveness as compared with a hydrogel including a poloxamer alone or a hydrogel outside of the above-described ranges.

Particularly, the bone graft composition of the present invention may have a yield value ranging from 1500 g/cm² to 4000 g/cm². When the composition of the present invention has a yield value within the above-described range, it will exhibit excellent viscoelasticity, and thus may be easily filled in bone defects during grafting, suggesting that it shows physical properties suitable for use as a graft material.

As used herein, the term "compressive strength" is used interchangeably with "strength", and means the strength at which the external shape of the bone graft composition is changed by an external force. The term "yield value" is a physical property value related to the elasticity of a finished product, and means the maximum strength at which the composition is not deformed by an external force. Thus, as the compressive strength and yield value increase, it is possible to maintain the abilities of the composition to fix its location and maintain its external shape in the closure or the implant placement stage after injection or dense filling of the bone graft material.

As used herein, the term "adhesiveness" means the property of adhering to stainless steel. It is a force acting in a direction opposite to that of compressive strength, and (-) means only direction. The higher absolute value of the adhesiveness means that a greater force is required to detach the bone graft composition formulation that stuck to stainless steel, and it may also mean the degree of adhesion of the bone graft composition formulation not only to a surgical tool made of stainless steel, but also to gloves made of resin.

Compressive strength, yield value, and adhesiveness, which are physical strengths that are measured in the present invention, may be measured using a common rheometer and/or UTM (universal testing machine).

The bone graft composition of the present invention includes a hydrogel filled between calcium phosphate compound particles close to each other. After implantation of the bone graft composition into bone defects, the hydrogel is degraded and released, the calcium phosphate compound particles are maintained in the close state, and bone grows into the space between the calcium phosphate compound particles after release of the hydrogel. Therefore, it is necessary to have the ability to maintain its shape for a predetermined period of time.

The bone graft composition of the present invention may further include a physiologically active substance. The physiologically active substance may be loaded in pores of the porous calcium phosphate compound particles included in the composition. The physiologically active substance may be one or more selected from the group consisting of bone morphogenetic proteins (BMPs), bone morphogenetic peptides, extracellular matrix proteins, and tissue growth factors.

For example, the bone morphogenetic proteins may include BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, or a combination thereof, but are not limited thereto.

For example, the tissue growth factors may include VEGF, FGF-2, IGF-1, TGF-β, or the like, but are not limited thereto.

The bone graft composition of the present invention is for use in bone grafting, maxillary sinus lifting, lumbar interbody fusion, cervical interbody fusion, or upper and lower extremity fracture fusion, but is not limited thereto. For example, the bone graft composition may be used in lateral lumbar interbody fusion (LLIF), oblique lumbar interbody fusion (OLIF), or anterior lumbar interbody fusion (ALIF), but is not limited thereto. In particular, the bone graft composition of the present invention is a putty formulation which may be injected regardless of the shape of the site to be transplanted, and has excellent strength, and thus may be usefully applied to the bone damage site having an unspecified shape.

The composition of the present invention may be provided in a putty formulation.

Another aspect of the present invention provides a kit for bone implantation, the kit including the bone graft composition and an injection tool.

The kit of the present invention may be a bone graft material of a putty formulation. Such a graft material of the putty formulation may be injected into a desired site by putting it in an injection tool such as a syringe, tube, *etc.* After injection, the bone graft material may be packed by a surgical tool so that it is densely filled into bone defects due to a viscoelastic property of the product.

To this end, the kit of the present invention may further include an injection tool, wherein the injection tool may include a mixing syringe or a vial transport device, but is not limited thereto.

As described above, the kit for bone implantation of the present invention may be used in bone grafting, maxillary sinus lifting, lumbar interbody fusion, cervical interbody fusion, or upper and lower extremity fracture fusion, *e.g.,* lateral lumbar interbody fusion (LLIF), oblique lumbar interbody fusion (OLIF), or anterior lumbar interbody fusion (ALIF), but is not limited thereto.

### Advantageous Effects of Invention

The composition of the present invention is designed to compensate for the disadvantages of the existing formulation, for which it is difficult to improve physical properties, by using a microparticle-type calcium phosphate-based compound and increasing the content thereof. When a bone graft material is prepared by mixing the calcium phosphate-based compound with hydrogel, a mixture of microparticles and macroparticles is used as the calcium phosphate-based compound to increase the content of the calcium phosphate-based compound by 70% or more based on the total composition. Thus, the composition may maintain its shape for a long period of time under *in vivo* mimetic conditions, and enables sustained release of a drug loaded therein, and therefore, it may be usefully applied as a graft material for regeneration of injured bone tissues by loading physiologically active substances such as bone morphogenetic proteins, *etc.*

### Brief Description of Drawings

FIG. 1 shows actual appearances of various sizes (r) of calcium phosphate-based compound particles;
FIG. 2 shows appearances of compositions prepared by mixing various sizes of calcium phosphate-based compound particles with hydrogel at predetermined ratios;
FIG. 3 shows an exemplary formulation of a composition according to one exemplary embodiment of the present invention, wherein the left shows the use of the composition filled in a putty-type syringe which is used in dentistry, and the right shows the use of the composition filled in a case which is used in spine fusion surgery;
FIG. 4 shows elasticity and/or texture of the composition according to one exemplary embodiment of the present invention;
FIG. 5 shows shape retention ability of bone graft materials under *in vivo* mimetic conditions, the bone graft materials composed of the compositions which were prepared by mixing various sizes of calcium phosphate-based compounds with hydrogel at predetermined ratios;
FIG. 6 shows sustained drug release of bone graft materials under *in vivo* mimetic conditions, the bone graft materials composed of the compositions which were prepared by mixing various sizes of calcium phosphate-based compounds with hydrogel at predetermined ratios;
FIG. 7 shows shear strain vs. shear stress of the bone graft material prepared according to one exemplary embodiment of the present invention in a fluid environment (black solid line), wherein as a control (blue solid line), a bone graft composition of Comparative Example 6 was used; and
FIG. 8 shows changes in compressive strength according to the sizes of the calcium phosphate compound particles of the bone graft materials prepared according to one exemplary embodiment of the present invention.

### Mode for the Invention

Hereinafter, the configuration and effects of the present invention will be described in more detail with reference to exemplary embodiments. However, these exemplary embodiments are for illustrative purposes only, and the scope of the present invention is not intended to be limited by these exemplary embodiments.

### Preparation Example 1: Preparation of calcium phosphate compound particle powder 1

Pure β-TCP powder (Cerectron Co., Korea) was spray-dried to prepare a spherical shape. Then, the spherical β-TCP powder was sintered at 1050°C, and the sintered particles were classified in the range of 45 µm to 75 µm.

### Preparation Example 2: Preparation of calcium phosphate compound particle powder 2

Calcium phosphate compound particles having a distribution in the range of 200 µm to 6,000 µm were prepared with reference to the method disclosed in Korean Patent No. 10-0401941.

### Example 1: Preparation of high-elasticity calcium phosphate-based bone graft material

First, HPMC and poloxamer 407 were mixed using a high-speed vacuum mixer to produce hydrogel, and then the β-TCP powder prepared according to Preparation Example 1 was uniformly mixed therewith to obtain a hydrogel complex.

Subsequently, the prepared hydrogel complex was mixed with the hydroxyapatite ceramic granules having a size of 0.6 mm to 6 mm prepared according to Preparation Example 2 to prepare a bone graft material of a putty formulation. The mixing was performed using a specialized mixing syringe so that the hydroxyapatite granules were pulverized.

### Comparative Examples 1 to 4: Various sizes of calcium phosphate-based compound particles

Particle-type calcium phosphate-based compounds having a size in the range of less than 100 µm, 600 µm to less than 1,000 µm, 1,000 µm to less than 3,000 µm, and 3,000 µm to 6,000 µm were prepared using the samples of Comparative Examples 1 to 4, respectively.

### Comparative Examples 5 to 7: Bone graft composition including controlled contents of calcium phosphate-based compound microparticles and hydrogel

Calcium phosphate-based compound particles having a size of less than 100 µm and hydrogel were mixed at a weight ratio of 30:70, 50:50, and 70:30 to prepare bone graft compositions of Comparative Examples 5 to 7, respectively.

### Comparative Example 8: Bone graft composition including controlled contents of calcium phosphate-based compound macroparticles and hydrogel

Calcium phosphate-based compound particles having a size of 1,000 µm to less than 3,000 µm and hydrogel were mixed at a weight ratio of 50:50 to prepare a bone graft composition of Comparative Example 8.

### Experimental Example 1: Appearance and physical properties of bone graft composition

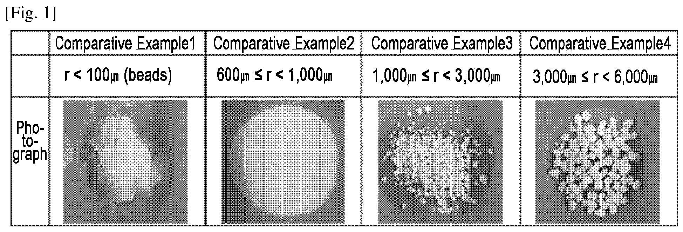

The shapes of the calcium phosphate-based compound particles of Comparative Examples 1 to 4 were observed with the unaided eye and photographed, and are shown in FIG. 1. As shown in FIG. 1, when the calcium phosphate-based compounds were used alone, it was difficult to process the compounds into a desired shape because they were composed of particles, and thus their use as a bone graft material was very limited.

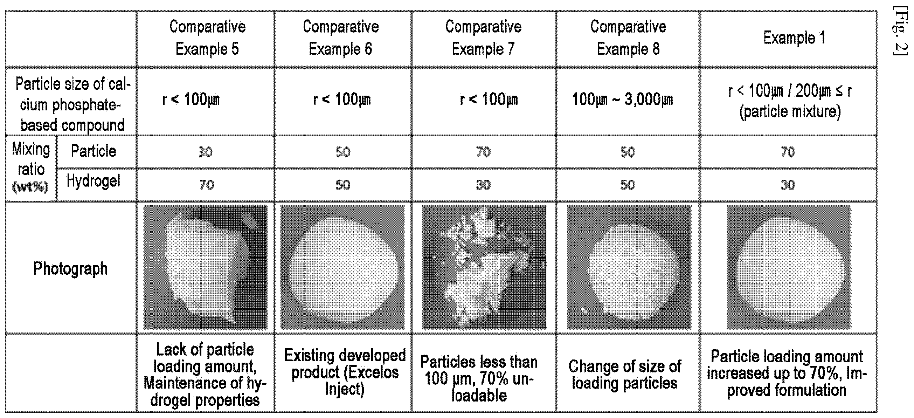

Further, appearance and features of the bone graft compositions of Comparative Examples 5 to 8 and Example 1 are shown in FIG. 2. As shown in FIG. 2, the bone graft compositions composed of calcium phosphate-based compound particles having a size of less than 100 µm and hydrogel showed the difference in appearance according to the composition ratio of these components, which was visible to the unaided eye. When the content of the calcium phosphate-based compound was as low as 30% (Comparative Example 5), the particle loading amount was insufficient, and thus the physical properties closer to the hydrogel were maintained. However, when the content of the calcium phosphate-based compound was 50% (Comparative Example 6, Excelos Inject), particles agglomerated well with the hydrogel to form and maintain a clay-like shape, similar to Example 1, when examined with the unaided eye. Meanwhile, in the composition (Comparative Example 8), which was prepared by mixing calcium phosphate-based compound particles having a size of 1,000 µm to less than 3,000 µm and hydrogel at a weight ratio of 50:50, although the particles agglomerated into a single mass, a structure with a rough surface was formed due to individual particles. Furthermore, as in the composition of Example 1, when the content of the calcium phosphate-based compound was increased by 70% (Comparative Example 7), only microparticles with a size of less than 100 µm could not agglomerate into a single mass and crumbled due to the excessive loading amount of particles. Meanwhile, as in Comparative Example 7, even though the content of the calcium phosphate-based compound was as high as 70%, when macroparticles having a size of 200 µm or more were further included in addition to calcium phosphate-based compound microparticles having a size of less than 100 µm, they were found to aggregate with the hydrogel to form a single mass, as in Comparative Example 6. This suggests that a bone graft composition having a higher content of calcium phosphate compound may be provided by using a mixture of microparticles and macroparticles.

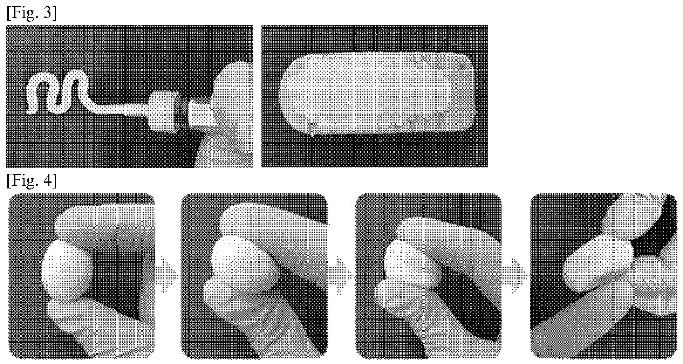

As described, the bone graft composition of Example 1, which was prepared by including the high 70% content of the calcium phosphate-based compound, was formulated into various preparations, and the clinical applicability thereof was tested. The test results are shown in FIGS. 3 and 4. As shown in FIG. 3, the bone graft composition of Example 1 could be injected using a dental putty-type syringe, and it was easily filled in a cage used for spinal fusion. Further, as shown in FIG. 4, when the shape was deformed by pressing it even with fingers, the composition could be easily deformed into a desired shape and did not stick to the fingers, and it was advantageous in controlling, with no concern about loss. As described above, since the bone graft composition of the present invention has enough fluidity to be injected using a syringe, it may be directly injected into a defect where it is difficult to accomplish a desired shape. It is also easy to obtain a desired shape by hand or using a predetermined cast, and the corresponding shape may be maintained. Thus, it may be used for bone regeneration.

### Experimental Example 2: Shape retention ability and sustained drug release under in vivo mimetic conditions

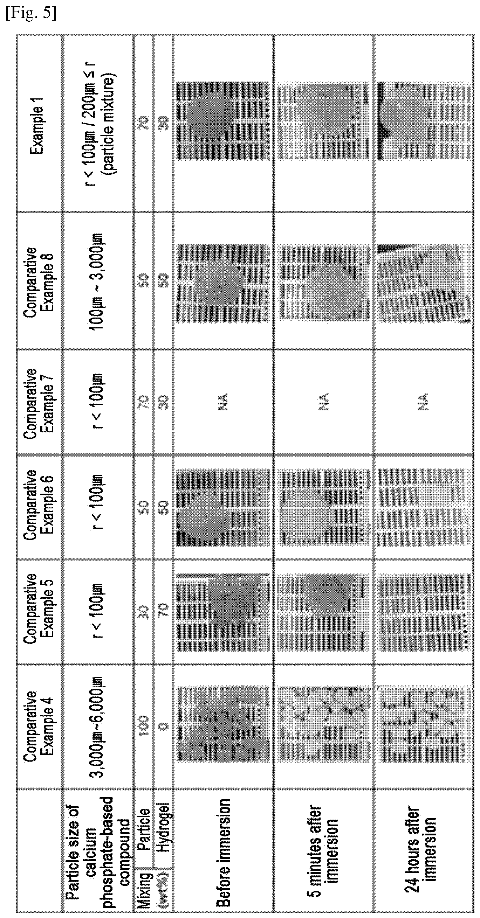

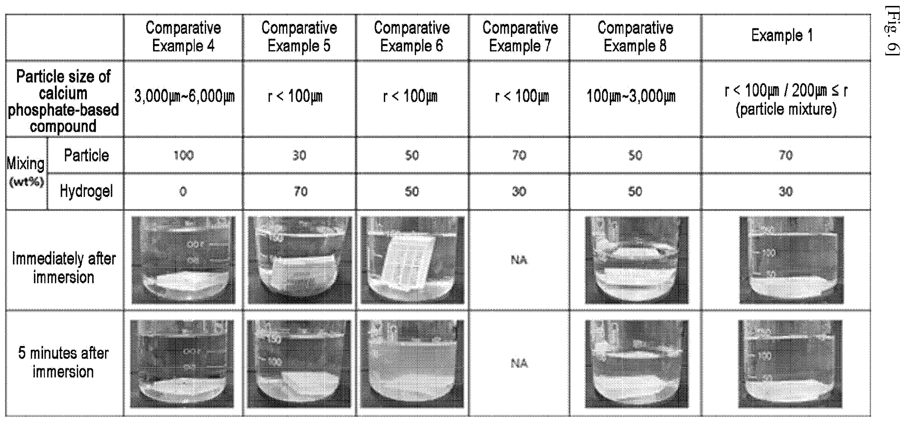

To examine properties of the bone graft materials under *in vivo* mimetic conditions, each of the compositions of Comparative Examples 4 to 8 and Example 1 was put in a cage and immersed in physiological saline at 37°C. After 5 minutes and 24 hours of immersion, their shape retention was examined. The results are shown in FIG. 5. Furthermore, in order to examine the release patterns of the bone graft materials when a drug was loaded therein, a red dye was loaded instead of the drug so that each sample was visually identified and then treated as described above. The color of the solution was examined before immersing in physiological saline and after 5 minutes of immersion, and the results are shown in FIG. 6.

As shown in FIG. 5, when the macroparticle-type calcium phosphate-based compound was used alone (Comparative Example 4), even at 24 hours after being immersed in physiological saline similar to the body temperature, no change in the shape was visually observed. When the content of the calcium phosphate-based compound was as low as 30% (Comparative Example 5), the shape was actually maintained until 5 minutes after being immersed in physiological saline, but after 24 hours, it was completely decomposed, and the shape could not be identified. This indicates that no strong physical bond was formed between the calcium phosphate-based compound particles and the hydrogel. When the content of the calcium phosphate-based compound was 50% (Comparative Example 6, Excelos Inject), an almost intact shape was maintained until 5 minutes after being immersed in physiological saline. However, after 24 hours, most of the particles were decomposed and only partly remained. This indicates that the particles still failed to form a bond strong enough to withstand the *in vivo* mimetic conditions. Meanwhile, when the content of the calcium phosphate-based compound was as high as 70% (Comparative Example 7), the desired shape could not be obtained because the particles were not sufficiently combined as they are, and thus additional experiments were not possible, as confirmed in Experimental Example 1. Furthermore, the composition including 50% of the calcium phosphate-based compound macroparticles (Comparative Example 8) exhibited excellent shape retention ability, as compared with the composition of Comparative Example 6 including the same amount of the microparticles, but a significant portion thereof was decomposed. In contrast, the composition of Example 1, which was prepared by including 70% of both the microparticles and the macroparticles, maintained the existing shape without structural decomposition even after 24 hours. This suggests that the graft material composed of the composition of Example 1 is a material suitable for use as a bone graft material, which is able to maintain its structure even in a practical surgical environment such as bone implantation and/or spinal fusion.

As shown in FIG. 6, the graft materials of Comparative Examples 4 to 6 and 8, excluding Comparative Example 7, in which experimentation was impossible, began to release the dye which was clear enough to be identified with the unaided eye, immediately after being immersed in physiological saline, and after 5 minutes, most dye was released. In detail, as compared with Comparative Example 4, in which the calcium phosphate-based compound particles were used alone, when a predetermined amount of hydrogel was included, the degree was slightly reduced. In particular, in the composition of Comparative Example 5 including the hydrogel at as high as 70%, initial release was considerably inhibited, indicating that diffusion of the dye was physically inhibited by the hydrogel. In contrast, the graft material of Example 1 released a small amount of dye, but the degree was insignificant, and the released amount was significantly smaller than the results of other Comparative Examples. This result was also confirmed from FIG. 5, showing that the color of the graft material became pale. Taken together, in the composition of Example 1, even though the content of hydrogel was slightly low at 30%, the release of the dye was overall inhibited, suggesting that when a drug is loaded in the composition of Example 1, sustained release thereof may be achieved.

### Experimental Example 3: Comparison of yield stress in fluid

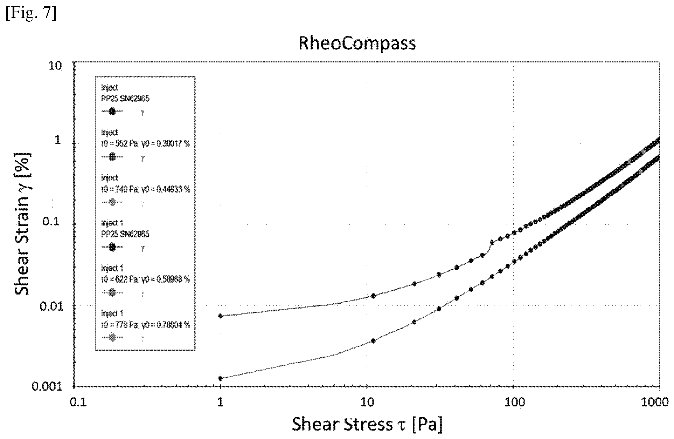

The same force (shear stress) was applied using a rheometer to the graft material composed of the composition of Comparative Example 6, which is a commercially available product, and the graft material composed of the composition of Example 1, in which calcium phosphate-based compound macroparticles were additionally included to increase the content thereof. At this time, the shear strain of each formulation was measured and shown in FIG. 7. As shown in FIG. 7, it was confirmed that when the same force was applied, the deformation of the control group was more severe than that of the specimen of Example 1. This indicates that the formulation of Example 1 is able to maintain its shape without structural decomposition, when in contact with water or blood flow in the *in vivo* environment in which it is implanted, that is, when a physical force is applied, and therefore, a drug, *e.g.,* BMP-2, loaded therein may be sustained-released.

### Experimental Example 4: Comparison of strength according to particle size of calcium phosphate compound

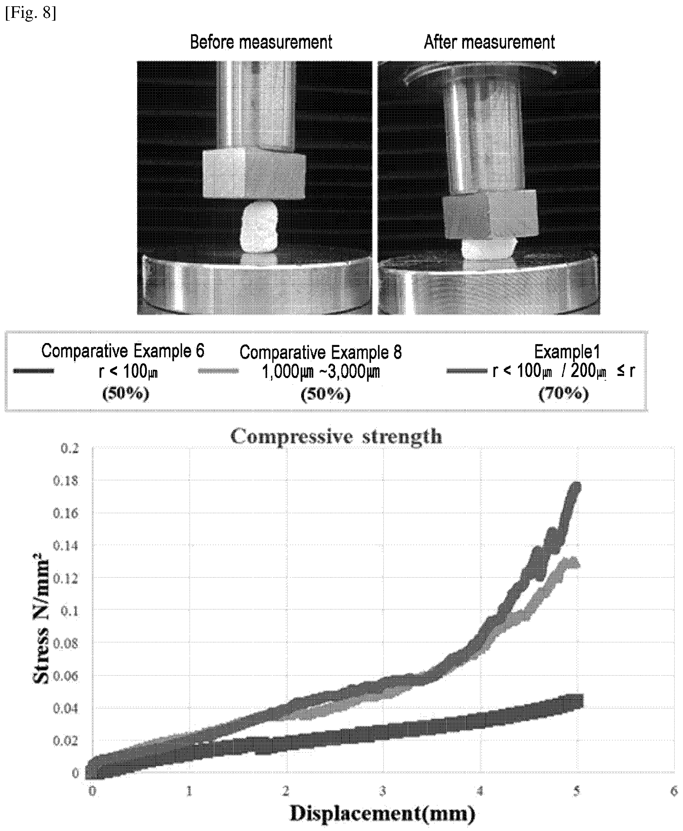

In order to examine changes in the strength of the composition of Example 1 in which the content of calcium phosphate-based compound was increased by additionally including macroparticles in addition to calcium phosphate-based compound microparticles, the composition of Example 1 in which the content of the calcium phosphate-based compound was increased to 70% by including both microparticles and macroparticles, the composition of Comparative Example 6 in which the content of the calcium phosphate-based compound was 50% by including only microparticles, and the composition of Comparative Example 8 in which the content of the calcium phosphate-based compound was 50% by including only macroparticles were measured for compressive strength, and the results are shown in FIG. 8. In detail, specimens of 8 mm×10 mm in size were prepared by using each composition, and deformation under compression was measured, as shown at the top of FIG. 8. As shown in FIG. 8, when Comparative Examples 6 and 8 were compared with each other, Comparative Example 8 showed remarkably improved strength, in which the composition had the same content, but the particle had the larger size. In contrast, the composition of Example 1 including both microparticles and macroparticles showed improved strength due to the increased content thereof, because even though microparticles were included, they were mixed with the macroparticles, as compared to that of Comparative Example 8.

The scope of the disclosure is defined by the appended claims.

## Claims

1. An injectable bone graft composition comprising:
more than 55wt% and 80wt% or less of calcium phosphate compound particles; and
20wt% or more and less than 45wt% of biodegradable hydrogel,
wherein the calcium phosphate compound particles are a mixture of porous particles having a size of 45 µm to 100 µm, and 200 µm to 6,000 µm in mean diameter

2. The injectable bone graft composition of claim 1, wherein the calcium phosphate compound is any one or a combination of two or more selected from the group consisting of hydroxyapatite, tricalcium phosphate (TCP, Ca₃(PO₄)₂), tetracalcium phosphate (Ca₄(PO₄)₂O), brushite (CaHPO₄·2H₂O), dicalcium diphosphate (Ca₂P₂O₇), calcium tripolyphosphate (Ca₅(P₃O₁₀)₂), Mg-containing apatite, Mg-containing tricalcium phosphate, Sr-containing apatite, and fluorapatite.

3. The injectable bone graft composition of claim 1, wherein the porous particles have porosity of 60vol% (% by volume) or more.

4. The injectable bone graft composition of claim 1, wherein the hydrogel includes one or more selected from the group consisting of a poloxamer, collagen, hyaluronic acid, gelatin, a PEG/PPG/PEG block copolymer, and cellulose.

5. The injectable bone graft composition of claim 4, wherein the hydrogel is a material having a non-crosslinked structure without a swelling property.

6. The injectable bone graft composition of claim 1, further comprising a physiologically active substance.

7. The injectable bone graft composition of claim 6, wherein the physiologically active substance is one or more selected from the group consisting of bone morphogenetic proteins, bone morphogenetic peptides, extracellular matrix proteins, and tissue growth factors.

8. The injectable bone graft composition of claim 1 for use in the following treatments: in bone grafting, maxillary sinus lifting, lumbar interbody fusion, cervical interbody fusion, or upper and lower extremity fracture fusion.

9. The injectable bone graft composition of claim 1, wherein the injectable bone graft composition is a putty formulation.

10. A kit for bone implantation, the kit comprising the bone graft composition of any one of claims 1 to 7 and 9 and an injection tool.

11. The kit of claim 10, wherein the injection tool includes a mixing syringe or a vial transport device.

## Patentansprüche

1. Eine injizierbare Knochentransplantationszusammensetzung, umfassend:
mehr als 55 Gew.% und 80 Gew.% oder weniger
Calciumphosphatverbindungspartikel; und
20 Gew.% oder mehr und weniger als 45 Gew.% biologisch abbaubares Hydrogel, wobei die Calciumphosphatverbindungspartikel eine Mischung aus porösen Partikeln mit einer Größe von 45 µm bis 100 µm und einem mittleren Durchmesser von 200 µm bis 6.000 µm sind.

2. Die injizierbare Knochentransplantationszusammensetzung nach Anspruch 1, wobei die Calciumphosphatverbindung eine oder eine Kombination aus zwei oder mehr Verbindungen ist, die aus der Gruppe ausgewählt sind, bestehend aus Hydroxyapatit, Tricalciumphosphat (TCP, Ca₃(PO₄)₂), Tetracalciumphosphat (Ca₄(PO₄)₂O), Brushit (CaHPO₄·2H₂O), Dicalciumdiphosphat (Ca₂P₂O₇), Calciumtripolyphosphat (Ca₅(P₃O₁₀)₂), Mg-haltigem Apatit, Mg-haltigem Tricalciumphosphat, Sr-haltigem Apatit und Fluorapatit.

3. Die injizierbare Knochentransplantationszusammensetzung nach Anspruch 1, wobei die porösen Partikel eine Porosität von 60 Vol.% (Volumenprozent) oder mehr aufweisen.

4. Die injizierbare Knochentransplantationszusammensetzung nach Anspruch 1, wobei das Hydrogel eines oder mehrere aus der Gruppe bestehend aus einem Poloxamer, Kollagen, Hyaluronsäure, Gelatine, einem PEG/PPG/PEG-Blockcopolymer und Cellulose umfasst.

5. Die injizierbare Knochentransplantationszusammensetzung nach Anspruch 4, wobei das Hydrogel ein Material mit einer nicht-quervernetzten Struktur ohne Quellfähigkeit ist.

6. Die injizierbare Knochentransplantationszusammensetzung nach Anspruch 1, weiterhin umfassend eine physiologisch aktive Substanz.

7. Die injizierbare Knochentransplantationszusammensetzung nach Anspruch 6, wobei die physiologisch aktive Substanz eine oder mehrere Substanzen ist, die aus der Gruppe ausgewählt sind, bestehend aus knochenmorphogenetischen Proteinen, knochenmorphogenetischen Peptiden, extrazellulärenmean Matrix-Proteinen und Gewebewachstumsfaktoren.

8. Die injizierbare Knochentransplantationszusammensetzung nach Anspruch 1 zur Verwendung bei folgenden Behandlungen: bei Knochentransplantation, maxillarem Sinuslift, lumbaler Wirbelkörperfusion, zervikaler Wirbelkörperfusion oder Fusion von Frakturen der oberen und unteren Extremitäten.

9. Die injizierbare Knochentransplantationszusammensetzung nach Anspruch 1, wobei die injizierbare Knochentransplantationszusammensetzung eine knetbare Formulierung ist.

10. Ein Kit für die Knochenimplantation, wobei das Kit die Knochentransplantationszusammensetzung nach einem der Ansprüche 1 bis 7 und 9 und ein Injektionswerkzeug umfasst.

11. Das Kit nach Anspruch 10, wobei das Injektionswerkzeug eine Mischspritze oder eine Vial-Übertragungsvorrichtung umfasst.

## Revendications

1. - Composition injectable de greffe osseuse comprenant :
- plus de 55 % en poids et 80 % en poids ou moins de particules de composé de phosphate de calcium ; et
- 20 % en poids ou plus et moins de 45 % en poids d'hydrogel biodégradable,
dans laquelle les particules de composé de phosphate de calcium sont un mélange de particules poreuses ayant une taille de 45 *µ*m à 100 *µ*m, et un diamètre moyen de 200 *µ*m à 6 000 *µ*m.

2. - Composition injectable de greffe osseuse selon la revendication 1, dans laquelle le composé de phosphate de calcium est l'un quelconque ou une combinaison d'au moins deux choisis dans le groupe constitué par l'hydroxyapatite, le phosphate tricalcique (TCP, Ca₃(PO₄)₂), le phosphate tétracalcique (Ca₄(PO₄)₂O), la brushite (CaHPO₄.2H₂O), le diphosphate dicalcique (Ca₂P₂O₇), le tripolyphosphate de calcium (Cas (P₃O₁₀)₂), l'apatite contenant du Mg, le phosphate tricalcique contenant du Mg, l'apatite contenant du Sr et la fluorapatite.

3. - Composition injectable de greffe osseuse selon la revendication 1, dans laquelle les particules poreuses ont une porosité de 60 vol% (% en volume) ou plus.

4. - Composition injectable de greffe osseuse selon la revendication 1, dans laquelle l'hydrogel comprend au moins l'un choisi dans le groupe consistant en un poloxamère, le collagène, l'acide hyaluronique, la gélatine, un copolymère à blocs PEG/PPG/PEG et la cellulose.

5. - Composition injectable de greffe osseuse selon la revendication 4, dans laquelle l'hydrogel est un matériau ayant une structure non réticulée sans propriété de gonflement.

6. - Composition injectable de greffe osseuse selon la revendication 1, comprenant en outre une substance physiologiquement active.

7. - Composition injectable de greffe osseuse selon la revendication 6, dans laquelle la substance physiologiquement active est au moins l'une choisie dans le groupe consistant en les protéines morphogénétiques osseuses, les peptides morphogénétiques osseux, les protéines de matrice extracellulaire et les facteurs de croissance tissulaire.

8. - Composition injectable de greffe osseuse selon la revendication 1 pour une utilisation dans les traitements suivants : greffe osseuse, élévation du sinus maxillaire, fusion intersomatique lombaire, fusion intersomatique cervicale, ou fusion des fractures des extrémités supérieures et inférieures.

9. - Composition injectable de greffe osseuse selon la revendication 1, dans laquelle la composition injectable de greffe osseuse est une formulation de mastic.

10. - Kit d'implantation osseuse, le kit comprenant la composition de greffe osseuse selon l'une quelconque des revendications 1 à 7 et 9 et un outil d'injection.

11. - Kit selon la revendication 10, dans lequel l'outil d'injection comprend une seringue de mélange ou un dispositif de transport de flacon.
